# EUROPEAN PATENT SPECIFICATION

(11) **EP 2 419 109 B1**
(45) Date of publication and mention of the grant of the patent: **13.04.2016**
(21) Application number: 10714246.5
(22) Date of filing: 13.04.2010
(51) Int. Cl.: A61K 9/20, A61K 31/57, A61P 15/18

(54) **METHOD FOR ON-DEMAND CONTRACEPTION USING LEVONORGESTREL OR NORGESTREL**
VERFAHREN ZUR BEDARFSWEISEN KONTRAZEPTION MIT LEVONORGESTREL ODER NORGESTREL
PROCÉDÉ DE CONTRACEPTION SUR DEMANDE UTILISANT LE LÉVONORGESTREL OU LE NORGESTREL

(30) Priority: 14.04.2009 US 169158 P
(43) Date of publication of application: 22.02.2012
(73) Proprietor: Laboratoire HRA-Pharma, 75003 Paris (FR); Family Health International, Durham, NC 27713 (US)
(72) Inventor: Ulmann André, 75005 Paris (FR); Gainer Eric, 75019 Paris (FR); Gray Raymond, Elizabeth, NewYor, NY 10005 (US)
(74) Representative: Chajmowicz, Marion
(86) International application number: PCT/EP2010/054813
(87) International publication number: WO 2010/119030

(56) References cited:
- WO-A1-2007/045513
- KOVACS G T ET AL: "A pre-coital pill? A preliminary in vitro study" BRITISH JOURNAL OF FAMILY PLANNING, PROFESSIONAL AND SCIENTIFIC PUBLICATIONS, LONDON, GB, vol. 26, no. 3, 1 January 2000 (2000-01-01), pages 165-166, XP009079794 ISSN: 0144-8625

## Description

The present invention relates to methods for on-demand contraception in a woman, especially women who do not have a regular sexual activity.

### Technical background of the invention:

Hormonal contraception is considered the most reliable method of reversible contraception today. It requires the continuous taking of pills, generally daily, regardless of the frequency of intercourses. For women with infrequent sexual intercourse, however, preparations that are dependent on coitus and thus can be taken less often, with reduced exposure to the effective ingredients, would be more advantageous. Although recognized for a long time (Canzler et al, Zbl. Gynäkol, 1984, 106:1182-1191), the need for such on-demand contraception remains unmet (Aitken et al, Contraception, 2008, 78:S28-S35).

Women are actually creating such methods themselves out of existing products. In Ghana, a study conducted in 2003 reported that women were using norethindrone tablets, marketed for treatment of various gynecologic problems, as an "on demand" oral contraceptive. More recently, anecdotal reports and data collected by colleagues at Family Health International indicate that women in other parts of Africa and elsewhere are deliberately using emergency contraceptive pills in this manner.

Although oral methods do not provide protection against sexually transmitted infections, studies conducted several decades ago reported that various doses of levonorgestrel used as a regular postcoital contraceptive may provide protection with an efficacy comparable to the overall efficacy of condoms and other barrier methods in typical use (United Nations Development Programme/ United Nations Population Fund/World Health Organization/World Bank Special Programme of Research, Development and Research Training in Human Reproduction, Task Force on Post-Ovulatory Methods of Fertility Regulation. Efficacy and side effects of immediate postcoital levonorgestrel used repeatedly for contraception. Contraception 2000;61:303-8). It was further proposed to evaluate whether a single vaginal administration of levonorgestrel gel prior to intercourse would interfere with the ovulatory process. (Brache et al, Contraception, 2007; 76:111-116).

International application WO2007045513 describes a method for contraception wherein a progestogen is administered transdermally prior to sexual intercourse. The administration can be performed with a transdermal precoital patch which is put before sexual intercourse and maintained during several days after intercourse.

Kovacs et al. (The British Journal of Family Planning, 2000:26(3):165-166) describes the results of a preliminary clinical study aiming at assessing the effect of levonorgestrel and norethisterone on cervical mucus.

### Summary of the invention:

The invention provides a method for contraception, which method comprises on-demand orally administering a dosage of 1.5 mg of levonorgestrel in a woman, within 24 hours before an intercourse.

### Detailed description of the invention:

The inventors propose that levonorgestrel could be used as a contraceptive which could be taken on-demand, rather than everyday as any hormonal contraceptive pill.

Levonorgestrel is D-(-)-ETHYL-13BETA ETHYNYL-17ALPHA HYDROXY-17 GONENE-4 ONE-3.

Levonorgestrel has been developed for regular contraception, as well as for postcoital emergency contraception.

### On-demand:

The subject or patient can be any human female. The invention provides an "on-demand contraception", which means that the woman may take levonorgestrel or norgestrel at any time when needed, i.e. when an intercourse is expected.

Preferably, the woman does not use any other hormonal contraceptive medication. In another preferred embodiment, the subject does not use any protection (condom, uterine device, spermicide, etc). Preferably no contraceptive is used after the intercourse.

The invention provides a method of contraception, which method comprises discontinuously administering levonorgestrel in a woman before an intercourse. According to the invention, the woman will not use levonorgestrel on a daily basis, and preferably not more than four days in a row. Preferably levonorgestrel is not administered more than four days, three or two days in a row.

### Routes of administration:

In the context of the invention, levonorgestrel is administered by oral route.

For a brief review of present methods for drug delivery, see, Langer, Science 249:1527-1533 (1990). Methods for preparing administrable compounds are known or are apparent to those skilled in the art and are described in more detail in, for example, Remington's Pharmaceutical Science, 17th Ed., Mack Publishing Company, Easton, Pa. (1985), and which is hereinafter referred to as "Remington."

Unit dosages of immediate-release formulations are preferred.

For solid compositions, conventional nontoxic solid carriers may be used which include, for example, pharmaceutical grades of mannitol, lactose, starch, magnesium stearate, sodium saccharine, talcum, cellulose, glucose, sucrose. For oral administration, a pharmaceutically acceptable nontoxic composition is formed by incorporating any of the normally employed excipients, such as those carriers previously listed.

Oral solid dosage forms are preferentially compressed tablets or capsules. Compressed tablets may contain diluents to increase the bulk of levonorgestrel or norgestrel so that production of a compressed tablet of practical size is possible. Binders, which are agents which impart cohesive qualities to powdered materials may bealso necessary. Povidone, starch, gelatin, sugars such as lactose or dextrose, and natural and synthetic gums may be used. Disintegrants are generally necessary in the tablets to facilitate break-up of the tablet. Disintegrants include starches, clays, celluloses, algins, gums and crosslinked polymers. Lastly small amounts of materials known as lubricants and glidants are included in the tablets to prevent adhesion of the tablet material to surfaces in the manufacturing process and to improve the flow characteristics of the powder material during manufacture. Colloidal silicon dioxide is most commonly used as a glidant and compounds such as talc, magnesium stearate or stearic acids are most commonly used as lubricants. Procedures for the production and manufacture of compressed tablets are well known by those skilled in the art (See Remington).

Capsules are solid dosage forms using preferentially either a hard or soft gelatin shell as a container for the mixture of levonorgestrel or norgestrel and inert ingredients. Procedures for production and manufacture of hard gelatin and soft elastic capsules are well known in the art (See Remington).

### Dosages

It is described that the unit dosage of levonorgestrel or norgestrel is more than 0.50mg, preferably between 0.50 and 1.50mg, preferably between 0.75 and 1.50mg.

Preferably the unit dosage of levonorgestrel is to be administered orally.

In the context of the invention, the unit dosage of levonorgestrel is 1.5 mg.

### Regimen:

Levonorgestrel can be administered within 24 hours *before* an intercourse, preferably within 12h before the intercourse, more preferably within 6h before the intercourse, preferably within 4h, preferably within 2h, still more preferably within 1h before the intercourse.

Levonorgestrel can be administered within 24 hours *before* an intercourse, and the same woman can also take levonorgestrel *after* the intercourse, preferably within 1h, also.

## Claims

1. A method for contraception, which method comprises on-demand orally administering a dosage of 1.5 mg of levonorgestrel in a woman, within 24 hours before an intercourse.

2. The method according to claim 1, wherein levonorgestrel is administered within 12h before the intercourse.

3. The method according to claim 1, wherein levonorgestrel is administered within 6h before the intercourse.

4. The method according to claim 1, wherein levonorgestrel is administered within 1h before the intercourse.

5. The method according to anyone of claims 1-4, wherein levonorgestrel is administered in the form of a tablet.

6. The method of anyone of claims 1-5, wherein levonorgestrel is administered in the form of an immediate-release dosage.

7. The method according to anyone of claims 1-6, wherein no contraceptive is used after the intercourse.

8. The method of anyone of claims 1-6, comprising orally administering levonorgestrel before and after the intercourse.

9. The method of anyone of claims 1-8 wherein levonorgestrel is further orally administered within 1 hour after intercourse.

10. The method according to anyone of claims 1-6 wherein the levonorgestrel is administered at most four days in a row.

## Patentansprüche

1. Ein Verfahren zur Empfängnisverhütung, wobei das Verfahren im Bedarfsfall die orale Verabreichung einer Dosis von 1,5 mg Levonorgestrel an eine Frau innerhalb von 24 Stunden vor dem Geschlechtsverkehr umfasst.

2. Das Verfahren nach Anspruch 1, wobei Levonorgestrel innerhalb von 12h vor dem Geschlechtsverkehr verabreicht wird.

3. Das Verfahren nach Anspruch 1, wobei Levonorgestrel innerhalb von 6h vor dem Geschlechtsverkehr verabreicht wird.

4. Das Verfahren nach Anspruch 1, wobei Levonorgestrel innerhalb lh vor dem Geschlechtsverkehr verabreicht wird.

5. Das Verfahren nach einem der Ansprüche 1-4, wobei Levonorgestrel in Form einer Tablette verabreicht wird.

6. Das Verfahren nach einem der Ansprüche 1-5, wobei Levonorgestrel in einer Darreichungsform mit sofortiger Wirkstofffreisetzung verabreicht wird.

7. Das Verfahren nach einem der Ansprüche 1-6, wobei nach dem Geschlechtsverkehr kein Empfängnisverhütungsmittel verwendet wird.

8. Das Verfahren nach einem der Ansprüche 1-6, umfassend die orale Verabreichung von Levonorgestrel vor und nach dem Geschlechtsverkehr.

9. Das Verfahren nach einem der Ansprüche 1-8, wobei Levonorgestrel darüber hinaus binnen 1 Stunde nach dem Geschlechtsverkehr oral verabreicht wird.

10. Das Verfahren nach einem der Ansprüche 1-6, wobei das Levonorgestrel höchstens vier Tage in Folge verabreicht wird.

## Revendications

1. Méthode de contraception, ladite méthode comprenant une administration orale à la demande d'une dose de 1,5 mg de lévonorgestrel à une femme, dans les 24 heures précédant un rapport sexuel.

2. Méthode selon la revendication 1, dans laquelle le lévonorgestrel est administré dans les 12 heures précédant le rapport sexuel.

3. Méthode selon la revendication 1, dans laquelle le lévonorgestrel est administré dans les 6 heures précédant le rapport sexuel.

4. Méthode selon la revendication 1, dans laquelle le lévonorgestrel est administré dans l'heure précédant le rapport sexuel.

5. Méthode selon l'une quelconque des revendications 1-4, dans laquelle le lévonorgestrel est administré sous la forme d'un comprimé.

6. Méthode selon l'une quelconque des revendications 1-5, dans laquelle le lévonorgestrel est administré sous la forme d'un dosage à libération immédiate.

7. Méthode selon l'une quelconque des revendications 1-6, dans laquelle aucun contraceptif n'est utilisé après le rapport sexuel.

8. Méthode selon l'une quelconque des revendications 1-6, comprenant l'administration orale de lévonorgestrel avant et après le rapport sexuel.

9. Méthode selon l'une quelconque des revendications 1-8, dans laquelle le lévonorgestrel est en outre administré oralement dans l'heure suivant le rapport sexuel.

10. Méthode selon l'une quelconque des revendications 1-6, dans laquelle le lévonorgestrel est administré au maximum quatre jours d'affilée.
